# EUROPEAN PATENT APPLICATION

(11) **EP 4 644 936 A1**
(43) Date of publication of application: **05.11.2025**
(21) Application number: 25173669.0
(22) Date of filing: 30.04.2025
(51) Int. Cl.: G01R 33/34, G01R 33/3415

(54) **RECEIVE COIL UNIT AND MAGNETIC RESONANCE IMAGING APPARATUS**

(30) Priority: 01.05.2024 JP 2024074347
(71) Applicant: FUJI-FILM Corporation, Minato-ku Tokyo 106-8620 (JP)
(72) Inventor: TAKIZAWA, Masahiro, Tokyo (JP)
(74) Representative: Dehns Germany Partnerschaft mbB

(57) **Abstract**

A receive coil unit having an apron shape includes a body part (11) in which a plurality of receive coils (3) that receive magnetic resonance signals from a subject (P) under examination are disposed, and that covers a front side and a lateral side of the subject under examination, a first strap-shaped part (13) and a second strap-shaped part (13) that each have one end portion connected to an upper portion of the body part and that have strap shapes respectively placed on both shoulders of the subject under examination, and a first belt (15) and a second belt (15) that are connected to the other end portion of the first strap-shaped part and the other end portion of the second strap-shaped part and that are attachable to and detachable from each other.

## Description

### BACKGROUND OF THE INVENTION

### 1. Field of the Invention

The present invention relates to a receive coil unit and a magnetic resonance imaging apparatus, and particularly to a receive coil unit having an apron shape that can be worn by a subject.

### 2. Description of the Related Art

In a magnetic resonance imaging (MRI) examination using an MRI apparatus, a radio frequency (RF) wave at a resonance frequency specific to an element is applied to an examination part of a subject, a generated nuclear magnetic resonance (NMR) signal is received by a receive coil (also referred to as an "RF coil"), and an image (MRI image) is generated from the received NMR signal. Therefore, the subject needs to wear the receive coil in order to receive the nuclear magnetic resonance signal at the examination part.

For example, in a case in which an abdomen of the subject is imaged by the magnetic resonance imaging apparatus, the examination technician attaches a receive coil unit for the abdomen to the subject after the subject lies on the examination bed of the magnetic resonance imaging apparatus.

Meanwhile, US9255977B discloses a receive coil unit that can be worn by being hung on one shoulder of a subject before the subject lies on an examination bed. The receive coil unit of US9255977B is hung from one shoulder such that the receive coils can be attached to a front side and a rear side of the subject before the subject lies on the examination bed.

### SUMMARY OF THE INVENTION

However, in a case in which an examination technician attaches the receive coils in a state in which the subject lies on the examination bed as described above, the examination technician has to move large receive coils from a storage rack with great care, which increases a burden on the examination technician.

In addition, in a case in which the subject wears the receive coils in a state of lying on the examination bed, the examination technician has to fix the receive coils with a fixing belt, and the burden on the examination technician increases. In addition, the examination technician has to connect a magnetic resonance imaging apparatus body and the receive coils with a connection cable, which increases the burden on the examination technician.

Further, in the receive coil unit of US9255977B, the receive coils are unstable to be worn by the subject since the receive coils are hang from one shoulder of the subject. In addition, in the receive coil unit of US9255977B, connection portions at two locations on one shoulder and the flank has to be provided, and there is a possibility that the subject may feel uncomfortable during the MRI examination.

The present invention has been made in view of such circumstances, and an object of the present invention is to provide a receive coil unit and a magnetic resonance imaging apparatus with which a burden on an examination technician in an MRI examination can be reduced, easy wearing can be achieved, and stable imaging with a magnetic resonance imaging apparatus can be performed.

In order to achieve the above-described object, a receive coil unit according to a first aspect of the present invention has an apron shape, the receive coil unit comprising: a body part in which a plurality of receive coils that receive magnetic resonance signals from a subject under examination are disposed, and that covers a front side and a lateral side of the subject under examination; a first strap-shaped part and a second strap-shaped part that each have one end portion connected to an upper portion of the body part and that have strap shapes respectively placed on both shoulders of the subject under examination; and a first belt and a second belt that are connected to the other end portion of the first strap-shaped part and the other end portion of the second strap-shaped part and that are attachable to and detachable from each other.

According to the aspect of the present invention, by configuring the receive coil unit having an apron shape with the body part, the first and second strap-shaped parts, and the first and second belts, it is possible to reduce the burden on the examination technician in the MRI examination, to achieve easy wearing, and to perform stable imaging with a magnetic resonance imaging apparatus.

In the receive coil unit according to a second aspect, it is preferable that the plurality of receive coils are disposed in the first strap-shaped part and the second strap-shaped part.

In the receive coil unit according to a third aspect, it is preferable that the first belt and the second belt are attachable to and detachable from each other by a hook-and-loop fastener.

In the receive coil unit according to a fourth aspect, it is preferable that a total weight of the first belt and the second belt is 20% or more of a weight of the body part.

In the receive coil unit according to a fifth aspect, it is preferable that the body part has a connector to which a reception cable connected to a magnetic resonance imaging apparatus is connected.

In the receive coil unit according to a sixth aspect, it is preferable that the connector is disposed at a position corresponding to a chest of the subject under examination.

In the receive coil unit according to a seventh aspect, it is preferable that the receive coil unit further comprise: an additional element that is connected to an end portion of the body part, that has the plurality of receive coils disposed therein, and that covers the front side and the lateral side of the subject under examination.

In the receive coil unit according to an eighth aspect, it is preferable that the receive coil unit further comprise: a fixing belt that is attached to the body part and that fixes the subject under examination to a table of an examination bed of a magnetic resonance imaging apparatus.

In the receive coil unit according to a ninth aspect, it is preferable that the receive coil unit further comprise: a winding belt for winding the body part around a body of the subject under examination.

A magnetic resonance imaging apparatus according to a tenth aspect comprises: the receive coil unit according to any one of the first to ninth aspects.

According to the aspects of the present invention, it is possible to reduce the burden on the examination technician in the MRI examination, and it is possible to perform a stable MRI examination without the subject feeling discomfort.

### BRIEF DESCRIPTION OF THE DRAWINGS

Fig. 1 is a perspective view showing an appearance of a magnetic resonance imaging apparatus (MRI apparatus).
Fig. 2 is a diagram showing a schematic configuration of an internal portion of the MRI apparatus shown in Fig. 1.
Fig. 3 is a conceptual diagram showing examination preparation steps of an MRI examination in the related art.
Fig. 4 is a conceptual diagram showing examination preparation steps of an MRI examination in a case in which a receive coil unit having an apron shape according to an embodiment of the present invention is used.
Fig. 5 is a diagram showing the receive coil unit having an apron shape.
Fig. 6 is a diagram showing a state in which a subject wears the receive coil unit having an apron shape, which is viewed from a lateral side.
Fig. 7 is a diagram showing a state in which the subject wears the receive coil unit having an apron shape, which is viewed from a rear side.
Fig. 8 is a diagram showing a receive coil unit having an apron shape according to a second embodiment.
Fig. 9 is a diagram showing a receive coil unit having an apron shape according to a third embodiment.
Fig. 10 is a diagram showing a receive coil unit having an apron shape according to a fourth embodiment.
Fig. 11 is a diagram showing a receive coil unit having an apron shape to which a plurality of additional elements are connected.
Fig. 12 is a diagram showing a receive coil unit having an apron shape according to a fifth embodiment.
Fig. 13 is a diagram showing a state in which the subject is fixed to a top plate.

### DESCRIPTION OF THE PREFERRED EMBODIMENTS

Hereinafter, preferred embodiments of a receive coil unit and a magnetic resonance imaging apparatus according to an embodiment of the present invention will be described with reference to the accompanying drawings.

### Appearance of MRI Apparatus

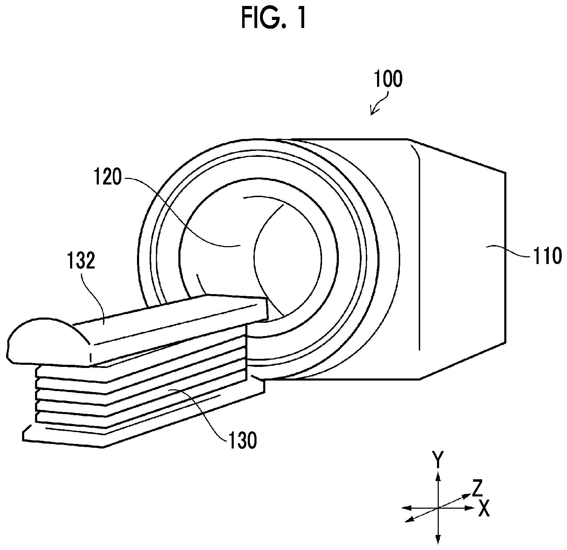

Fig. 1 is a perspective view showing an appearance of a magnetic resonance imaging apparatus (MRI apparatus). The receive coil unit according to the embodiment of the present invention is worn by a subject (subject under examination) in a case in which an MRI examination is performed with an MRI apparatus 100.

The MRI apparatus 100 shown in Fig. 1 comprises a gantry 110 and an examination bed 130 comprising a top plate (table) 132 disposed in front of a bore 120, which is an imaging space on a cylinder provided in the gantry 110.

### Internal Configuration of MRI Apparatus

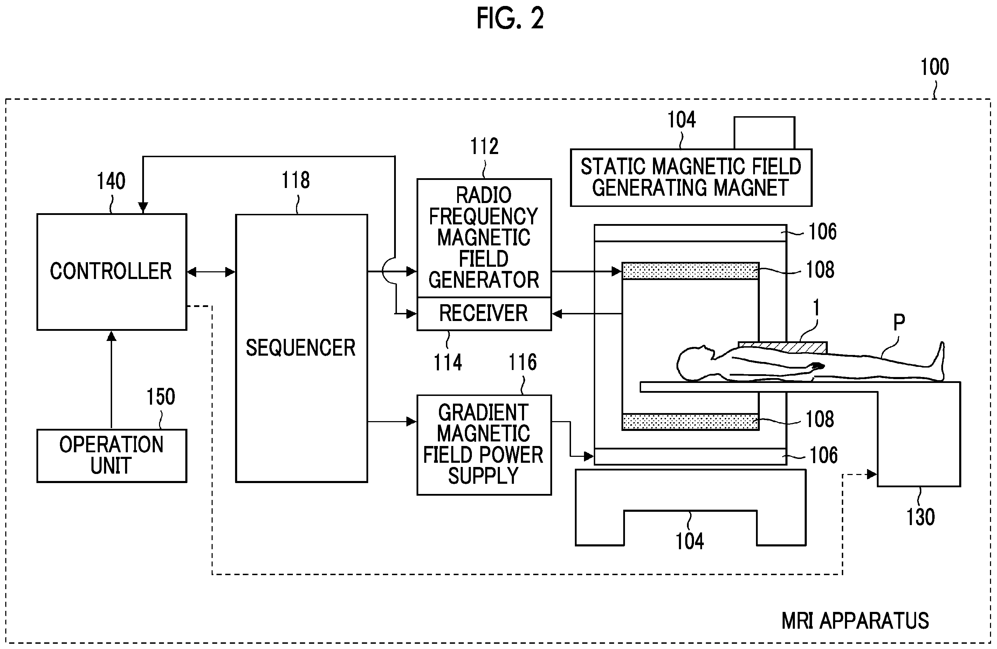

Fig. 2 is a diagram showing a schematic configuration of an internal portion of the MRI apparatus shown in Fig. 1.

As shown in Fig. 2, the MRI apparatus 100 comprises a static magnetic field generating magnet 104 that generates a uniform static magnetic field in the imaging space in which the subject P is disposed, a gradient magnetic field coil 106 that generates a gradient magnetic field in the imaging space, a radio frequency (RF) coil (transmit coil) 108 that generates a radio frequency magnetic field to cause a nucleus of an atom constituting a tissue of the subject P to generate a nuclear magnetic resonance signal (NMR signal), and a receive coil unit 1 comprising receive coils 3 that detect the NMR signal generated from the subject P.

The receive coil unit 1 is worn by the subject P before the subject P lies on the examination bed 130, and the subject P lies on the examination bed 130 after the subject P wears the receive coil unit 1. The subject P is usually disposed in the imaging space in a state of lying on the top plate 132 of the examination bed 130. It should be noted that the details of the receive coil unit 1 will be described later.

A sequencer 118 sends commands to a radio frequency magnetic field generator 112 and a gradient magnetic field power supply 116 in accordance with an imaging sequence (pulse sequence), to cause the generation of the radio frequency magnetic field and the gradient magnetic field, respectively. The generated radio frequency magnetic field is applied to the subject P as a pulsed radio frequency magnetic field (RF pulse) via the transmit coil 108. The NMR signals generated from the subject P are received by the receive coil unit 1.

It should be noted that the gradient magnetic field coil 106 is configured with gradient magnetic field coils in three directions, X, Y, and Z, and each of the gradient magnetic field coils generates the gradient magnetic field in response to a signal from the gradient magnetic field power supply 116.

The sequencer 118 controls each unit to operate at timing and intensity programmed in advance. Among programs, a program that particularly describes the timing and the intensity of the RF pulse, gradient magnetic field, and signal reception is called a pulse sequence.

Although various pulse sequences depending on the purpose are known, the detailed description thereof will be omitted here.

A controller 140 controls the operation of the MRI apparatus 100 via the sequencer 118 and performs various types of signal processing, such as image reconstruction.

The controller 140 can be configured with a computer. The computer applied to the controller 140 may be a personal computer or may be a workstation.

The controller 140 receives various instruction inputs from an operation unit 150 to perform overall control of each unit of the MRI apparatus 100 and to perform processing such as inverse Fourier transform of the echo signal in the spatial frequency region received from a receiver 114 to convert the echo signal into an image in a real space, thereby generating an MRI image while.

The operation unit 150 includes a mouse, a keyboard, and the like and functions as a part of a graphical user interface (GUI) that receives an input from an operator by using a display operation window of a display (not shown).

That is, the operation unit 150 and the display function as the GUI for the operator to input the start-up, the stop (temporary stop), the pulse sequence selection, the imaging conditions, the processing conditions, and the like of the MRI apparatus 100. In addition, the operation unit 150 according to the present example functions as an operation unit (operation button) used in a case in which the operator raises the examination bed 130 (top plate 132) at the start of the examination or in a case in which the subject pulls out the examination bed 130 (top plate 132) from the gantry 110 at the end of the examination. It should be noted that, in the example described in Fig. 2, the data obtained by the receive coil unit 1 (data including the NMR signal detected by the receive coils 3) is input to the receiver 114 via a reception cable K, subjected to AD conversion by the receiver, and then input to the controller 140. However, the present invention is not limited to this example. For example, the data may be wirelessly input to the controller 140. Specifically, the data obtained by the receive coil unit 1 may be subjected to AD conversion by an AD converter provided in the receive coil unit 1 and then transmitted to the controller 140 by a wireless device (radio wave or optical wireless) incorporated in the receive coil unit 1.

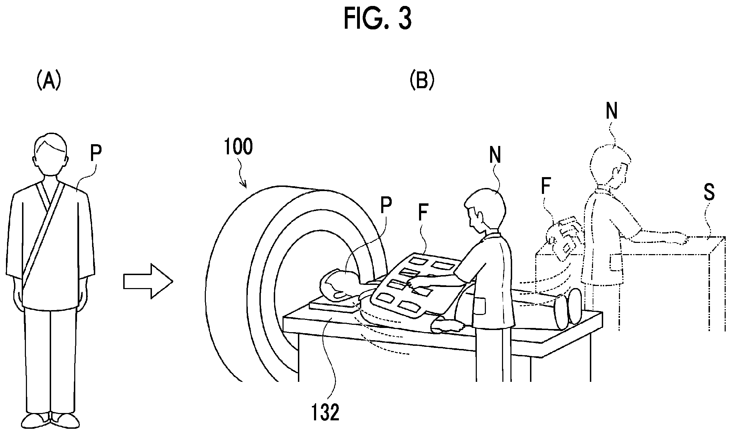

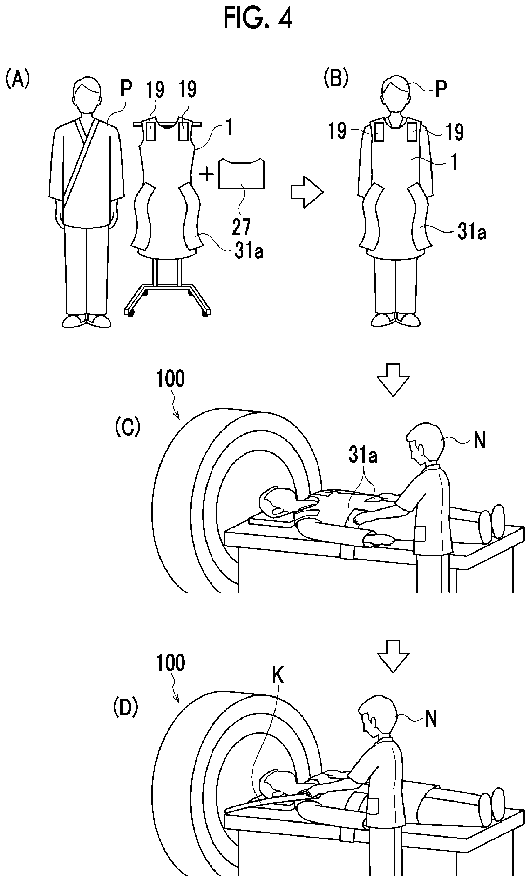

Next, preparation steps of the MRI examination using the MRI apparatus 100 will be conceptually described. Fig. 3 is a conceptual diagram showing examination preparation steps of an MRI examination in the related art. Fig. 4 is a conceptual diagram showing examination preparation steps of an MRI examination in a case in which the receive coil unit 1 having an apron shape according to the embodiment of the present invention is used.

First, the examination preparation steps of the MRI examination in the related art will be described.

(A) of Fig. 3 shows a step in which the subject P enters an examination room, (B) of Fig. 3 shows a step in which the subject P lies on the top plate 132 of the examination bed 130 of the MRI apparatus 100 and a receive coil unit F is attached. As described above, in a case in which the MRI examination is performed using the receive coil unit F in the related art, the receive coil unit F is attached by an examination technician N after the subject P lies on the top plate 132.

In this case, the examination technician N has to select and carry the receive coil unit F suitable for the subject P from a storage rack S in which a plurality of types of receive coil units and a plurality of sizes of receive coil units are stored. In addition, the examination technician N has to carefully attach a large receive coil unit F to the subject P after the subject P lies on the top plate 132. In addition, the examination technician N has to fix the subject P to the top plate 132 with a fixing belt (not shown in Fig. 3) that is a separate body from the receive coil unit F, and the handling of the fixing belt is a burden. In addition, the examination technician N has to connect the receive coil unit F to the MRI apparatus 100 with a reception cable (not shown in Fig. 3), and the handling of the connection cable is a burden. As described above, in the related art, there has been a heavy burden on the examination technician N in the preparation steps of the MRI examination.

Next, the examination preparation steps of the MRI examination in a case in which the receive coil unit 1 having an apron shape according to the embodiment of the present invention is used will be described.

(A) of Fig. 4 shows a step in which the subject P enters an examination room, (B) of Fig. 4 shows a step in which the subject P wears the receive coil unit 1 having an apron shape after entering and exiting the examination room, (C) of Fig. 4 shows a step in which the subject P is fixed to the top plate 132 with a fixing belt, and (D) of Fig. 4 shows a step in which the MRI apparatus 100 and the receive coil unit 1 are connected to each other with a reception cable. It should be noted that, although a fixing belt 31a, the receive coil unit 1 having an apron shape and having a connector 19, and an additional element 27 are shown in Fig. 4, these components will be described in detail later.

As shown in (A) and (B) of Fig. 4, the subject P enters the examination room and wears the receive coil unit 1 having an apron shape hanging on a dedicated rack by himself/herself. Thereafter, as shown in (C) of Fig. 4, the subject P who wears the receive coil unit 1 having an apron shape lies on the top plate 132 of the MRI apparatus 100. Then, the examination technician N fixes the subject P to the top plate 132 by the fixing belt 31a of the receive coil unit 1 having an apron shape. It should be noted that the fixing belt 31a is provided in the receive coil unit 1 having an apron shape as a part of the receive coil unit 1 having an apron shape.

Then, as shown in (D) of Fig. 4, the reception cable K connected to the MRI apparatus 100 is connected to the connector 19 provided at the corresponding position of the chest of the subject P.

As described above, in a case in which the receive coil unit is changed from the receive coil unit F in the related art to the receive coil unit 1 having the apron shape according to the embodiment of the present invention, it is possible to improve the examination preparation steps of the MRI examination. Specifically, by using the receive coil unit 1 having an apron shape, the examination technician N does not need to move the receive coil unit from the storage rack S, and the examination technician N does not need to move the receive coil unit after the subject P lies on the top plate 132 and attach the receive coil unit while paying attention. In addition, in a case in which the receive coil unit 1 having an apron shape has the fixing belt 31a, the examination technician N can fix the subject P to the top plate 132 without particularly handling the fixing belt 31a. In addition, in a case in which the receive coil unit 1 having an apron shape has the connector 19, the examination technician N can connect the reception cable K to the receive coil unit 1 having an apron shape without particularly needing to handle the reception cable K.

Next, the receive coil unit 1 having an apron shape will be described in detail.

### First Embodiment

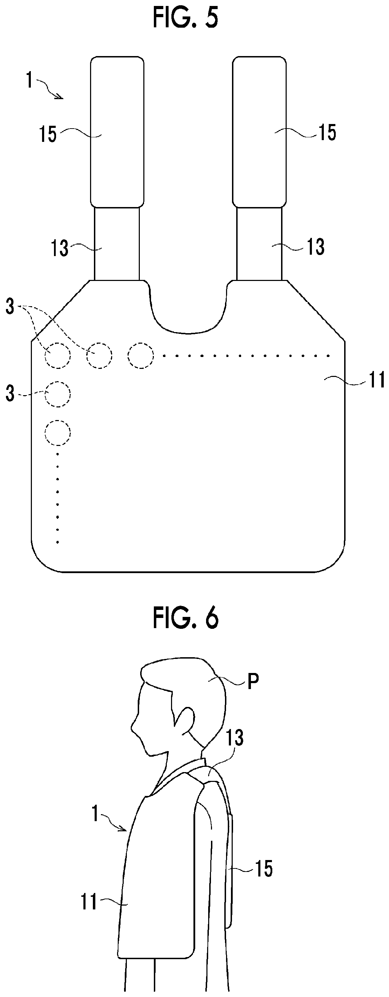

A first embodiment of the receive coil unit 1 having an apron shape will be described with reference to Figs. 5, 6, and 7.

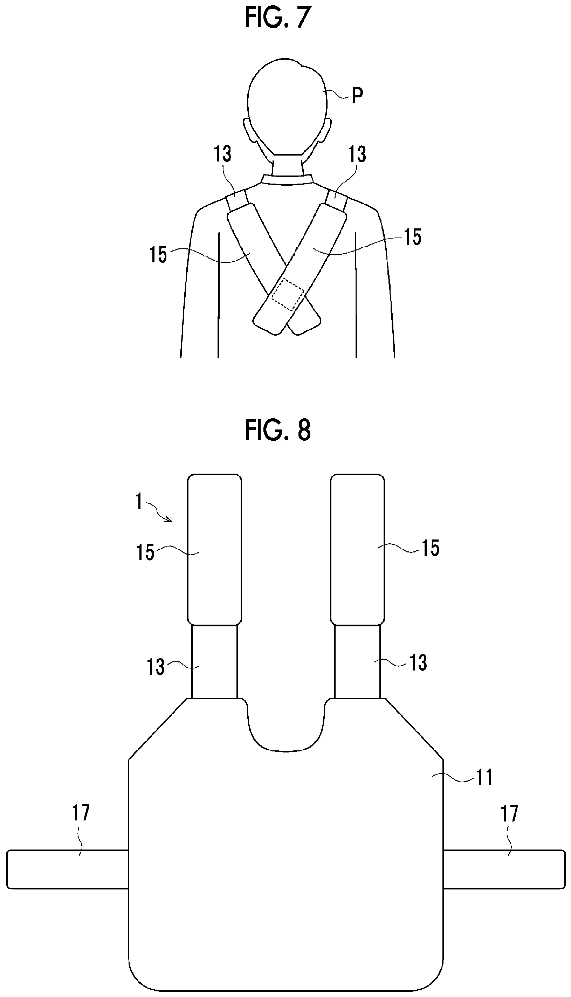

Fig. 5 is a diagram showing the receive coil unit 1 having an apron shape. Fig. 6 is a diagram showing a state in which the subject **P** wears the receive coil unit 1 having an apron shape, which is viewed from a lateral side. Fig. 7 is a diagram showing a state in which the subject P wears the receive coil unit 1 having an apron shape, which is viewed from a rear side.

As shown in Fig. 5, the receive coil unit 1 having an apron shape has an apron body part (body part) 11, shoulder parts (first strap-shaped part and second strap-shaped part) 13, and attachment belts (first belt and second belt) 15. The apron body part 11 has a plurality of receive coils 3, and the receive coils 3 receive the generated nuclear magnetic resonance (NMR) signals. In Fig. 5, although some receive coils 3 are shown and the other parts are omitted, the receive coils 3 are disposed on the entire surface of the apron body part 11. It should be noted that, in the following drawings, the description of the receive coils 3 is also omitted.

As shown in Fig. 6, the apron body part 11 is wide in a region (lateral region) corresponding to the armpit to the flank of the subject P, and can be bent along the flank of the subject P. As a result, it is possible to perform the MRI examination on the armpit and the flank of the subject P on which the receive coil unit 1 having the apron shape is attached.

The shoulder parts 13 are portions corresponding to both shoulders of the subject P, each have one end portion connected to an upper portion of the apron body part 11, have strap shapes respectively placed on both shoulders of the subject P, and are bent along the shoulder of the subject P as shown in Fig. 6. It is preferable that the shoulder parts 13 have a width enough to be a strap shape such that the shoulder parts 13 are stably hung on the shoulder. In addition, the plurality of receive coils 3 may be disposed in the shoulder parts 13, as in the apron body part 11. The MRI examination of the shoulder of the subject P can be performed by disposing the receive coils 3 at the shoulder parts 13.

The attachment belts 15 are crossed at the back of the subject P, and the receive coil unit 1 having an apron shape is attached to the subject P. The attachment belts 15 are attachable to and detachable from each other by a hook-and-loop fastener (magic tape (registered trademark)). As a result, the subject P does not have attachment fittings or the like on the back and can lie on the top plate 132 on the back. In addition, the attachment belts 15 are made to have a certain weight so that the weight of the attachment belts 15 are balanced with the weight of the apron body part 11 on the front side with the shoulder of the subject P as a fulcrum. For example, the total weight of the attachment belts 15 is 20% or more and 80% or less, and preferably 30% or more and 80% or less of the weight of the apron body part 11. The material of the attachment belts 15 is rubber or the like.

As described above, the subject P can wear the receive coil unit 1 having an apron shape by himself/herself, and easy wearing can be achieved by fastening the receive coil unit 1 at one place on the back. In addition, since the attachment belts 15 can be attached to and detached from each other by the hook-and-loop fastener, the metal fittings or the like are disposed on the back surface of the subject P and do not hinder the subject P from lying on his/her back.

### Second Embodiment

Next, a second embodiment of the receive coil unit 1 having an apron shape will be described. The receive coil unit 1 having an apron shape according to the present embodiment has winding belts 17 for winding around the subject P.

Fig. 8 is a diagram showing the receive coil unit 1 having an apron shape according to the second embodiment.

The receive coil unit 1 having an apron shape according to the present embodiment has an apron body part 11, shoulder parts 13, attachment belts 15, and winding belts 17.

The winding belts 17 are belts for winding the apron body part 11 around the body of the subject P. Specifically, the winding belts 17 can pass through the armpit of the subject P and can be fixed to the back. It is preferable that the winding belts 17 have a certain width such that the winding belts 17 are stably wound around the body of the subject P. It is preferable that the winding belts 17 are connected to each other by a hook-and-loop fastener (magic tape (registered trademark)).

As described above, since the receive coil unit 1 having an apron shape has the winding belts 17, the apron body part 11 is stably attached to the subject P, and a stable MRI examination can be performed.

### Third Embodiment

Next, a third embodiment of the receive coil unit 1 having an apron shape will be described. The receive coil unit 1 having an apron shape according to the present embodiment has a connector for connecting a reception cable.

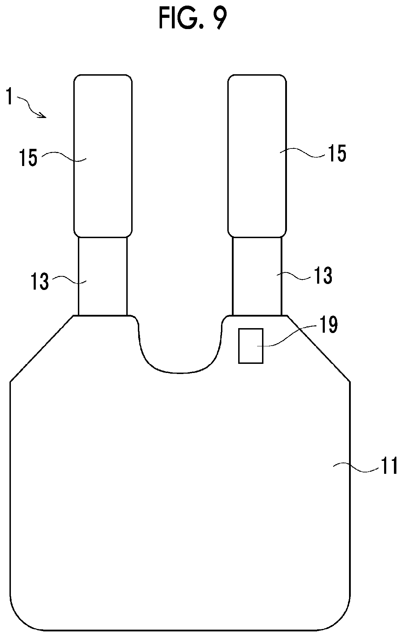

Fig. 9 is a diagram showing the receive coil unit 1 having an apron shape according to the third embodiment.

The receive coil unit 1 having an apron shape according to the present embodiment has an apron body part 11, shoulder parts 13, attachment belts 15, and a connector 19.

The connector 19 is provided at a position corresponding to the chest of the subject P of the apron body part 11. The connector 19 is configured with, for example, a socket, and MRI reception cable K connected to the MRI apparatus 100 is connected to the connector 19. As described above, by providing the connector 19 at the position corresponding to the chest of the subject P, it is possible to reduce the work of handling the reception cable that the examination technician N has to perform.

### Fourth Embodiment

Next, a fourth embodiment of the receive coil unit 1 having an apron shape will be described. In the receive coil unit 1 having an apron shape according to the present embodiment, the additional element 27 for size increase is j oined to an end portion of the apron body part 11. It should be noted that the receive coil unit 1 including the additional element 27 can transmit the data acquired by the additional element 27 (data including the NMR signals detected by the receive coils 3) from the apron body part 11 to the MRI apparatus 100 via, for example, the connector 19 and the reception cable K described in the third embodiment. In addition, in a case in which the AD converter and the wireless device are provided in the receive coil unit 1, the data can be wirelessly transmitted from the receive coil unit 1 to the MRI apparatus 100.

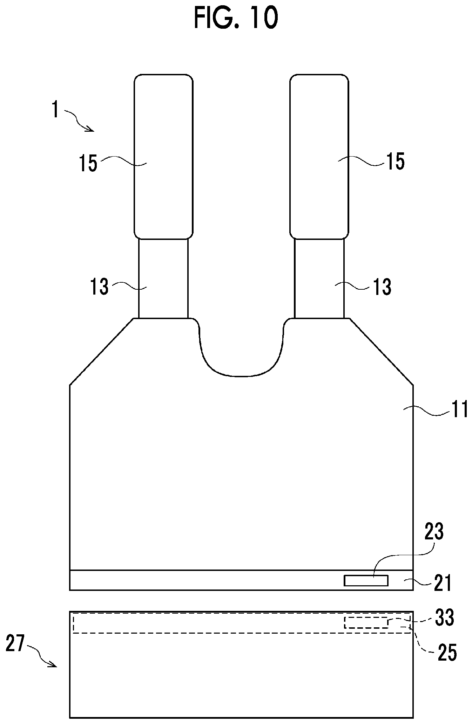

Fig. 10 is a diagram showing the receive coil unit 1 having an apron shape according to the fourth embodiment. The receive coil unit 1 having an apron shape according to the present embodiment has an apron body part 11, shoulder parts 13, attachment belts 15, and an additional element 27.

The additional element 27 is provided with the receive coils 3 in the same manner as the apron body part 11, and a location of the subject P covered with the additional element 27 can be targeted for the MRI examination. The additional element 27 can cover the front side and the lateral side of the subject P, similarly to the receive coil unit 1 having an apron shape. The additional element 27 is connected to an end portion of a lower portion of the apron body part 11. An overlapping region 21 is provided on the front surface of the end portion of the lower portion of the apron body part 11, and the overlapping region 21 comprises the connector 23 for connecting the receive coils 3 of the additional element 27. An overlapping region 25 is provided on the rear surface of the end portion of the upper portion of the additional element 27, and the overlapping region 25 has a connector 33 for connecting the receive coils 3 of the additional element 27 and the receive coils 3 of the apron body part 11. The overlapping region 21 of the apron body part 11 and the overlapping region 25 of the additional element 27 are connected by a hook-and-loop fastener.

As described above, by connecting the additional element 27 to the receive coil unit 1 having an apron shape, it is possible to increase the size, and it is possible to attach the receive coil unit corresponding to the size of the subject P.

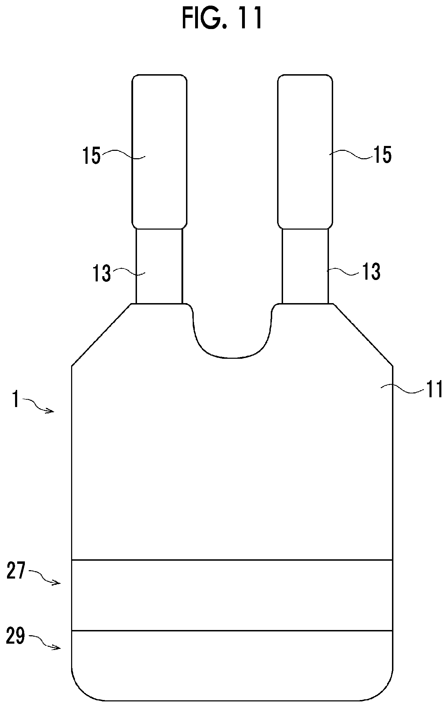

Fig. 11 is a diagram showing the receive coil unit 1 having an apron shape to which a plurality of additional elements are connected.

As shown in Fig. 11, the receive coil unit 1 having an apron shape can be connected to the plurality of additional elements. In the example shown in Fig. 11, the receive coil unit 1 having an apron shape connects the additional element 27 and an additional element 29. As described above, the size of the receive coil unit 1 having an apron shape can be changed to various sizes by adding the plurality of additional elements to the receive coil unit 1 having an apron shape. For example, the size adjustment is a small (S) size in a case in which only the receive coil unit 1 having an apron shape is provided, a medium (M) size in a case in which the additional element 27 is connected, and a large (L) size in a case in which the additional element 29 is further connected.

As described above, the plurality of additional elements are connected to the receive coil unit 1 having an apron shape, so that the receive coil unit 1 having an apron shape can be changed to various sizes.

### Fifth Embodiment

Next, a fifth embodiment of the receive coil unit 1 having an apron shape will be described. The receive coil unit 1 having an apron shape according to the present embodiment has fixing belts 31.

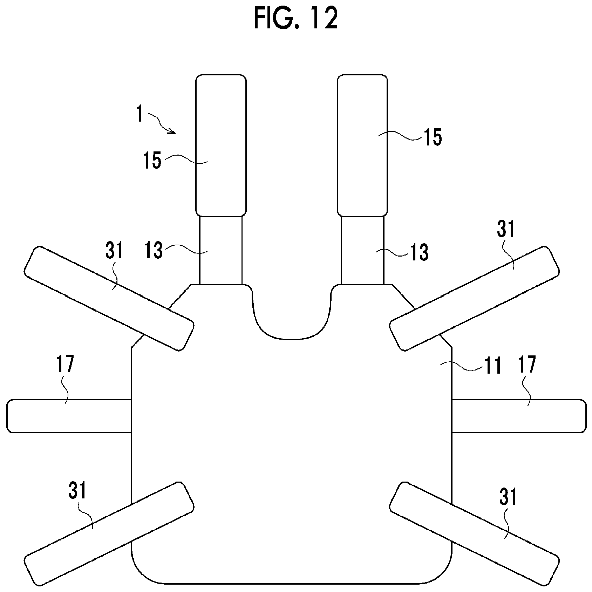

Fig. 12 is a diagram showing a receive coil unit having an apron shape 1 according to the fifth embodiment.

The receive coil unit 1 having an apron shape has an apron body part 11, shoulder parts 13, attachment belts 15, winding belts 17, and fixing belts 31.

The fixing belts 31 have a function of fixing the receive coil unit 1 having an apron shape to the top plate 132 on which the subject P lies. In the example shown in Fig. 12, the receive coil unit 1 having an apron shape has four fixing belts 31, and the fixing belts 31 are attached to the upper left, the upper right, the lower left, and the lower right. By fixing each of the four fixing belts 31 to the top plate 132, the subject P can be fixed to the top plate 132.

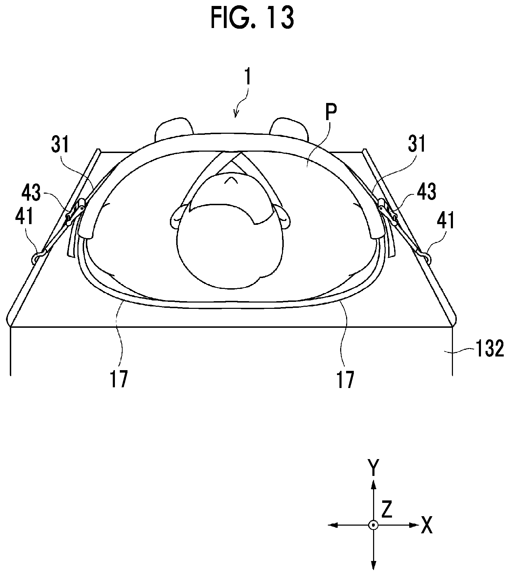

Fig. 13 is a diagram showing a state in which the subject P is fixed to the top plate 132.

The subject P lies on the top plate 132 on the back. Then, the subject P is fixed by the fixing belts 31. For example, the fixing belts 31 are fixed by hooking a hook-shaped coupler 41 to the top plate 132. In addition, for example, the length of the fixing belts 31 is adjusted by an adjustment mechanism 43 that adjusts the length of the fixing belts 31 after the fixing belts 31 are hooked on the top plate 132 by the coupler 41. It should be noted that a thin and soft material is preferably used for the fixing belts 31 so that the fixing belts 31 can be easily handled.

As described above, the receive coil unit 1 having the apron shape has the fixing belts 31, so that the subject P can be fixed on the top plate 132, and a stable MRI examination can be performed.

Although the examples of the present invention have been described above, it goes without saying that the present invention is not limited to the above-described embodiments, and various modifications can be made without departing from the gist of the present invention.

### Explanation of References

1: receive coil unit
3: receive coil
11: apron body part
13: shoulder part
15: attachment belt
17: winding belt
19: connector
21: region
23: connector
25: region
27: additional element
29: additional element
31: fixing belt
31a: fixing belt
33: connector
41: coupler
43: adjustment mechanism
100: MRI apparatus
N: examination technician
P: subject

## Claims

1. A receive coil unit having an apron shape, comprising:
a body part (11) in which a plurality of receive coils (3) that receive magnetic resonance signals from a subject (P) under examination are disposed, and that covers a front side and a lateral side of the subject under examination;
a first strap-shaped part (13) and a second strap-shaped part (13) that each have one end portion connected to an upper portion of the body part and that have strap shapes respectively placed on both shoulders of the subject under examination; and
a first belt (15) and a second belt (15) that are connected to the other end portion of the first strap-shaped part and the other end portion of the second strap-shaped part and that are attachable to and detachable from each other.

2. The receive coil unit according to claim 1,
wherein the plurality of receive coils are disposed in the first strap-shaped part and the second strap-shaped part.

3. The receive coil unit according to claim 1 or 2,
wherein the first belt and the second belt are attachable to and detachable from each other by a hook-and-loop fastener.

4. The receive coil unit according to any one of claims 1 to 3,
wherein a total weight of the first belt and the second belt is 20% or more of a weight of the body part.

5. The receive coil unit according to any one of claims 1 to 4,
wherein the body part has a connector (33) to which a reception cable connected to a magnetic resonance imaging apparatus (100) is connected.

6. The receive coil unit according to claim 5,
wherein the connector is disposed at a position corresponding to a chest of the subject under examination.

7. The receive coil unit according to any one of claims 1 to 6, further comprising:
an additional element (27) that is connected to an end portion of the body part, that has the plurality of receive coils disposed therein, and that covers the front side and the lateral side of the subject under examination.

8. The receive coil unit according to any one of claims 1 to 7, further comprising:
a fixing belt (31) that is attached to the body part and that fixes the subject under examination to a table of an examination bed of a magnetic resonance imaging apparatus (100).

9. The receive coil unit according to any one of claims 1 to 8, further comprising:
a winding belt (17) for winding the body part around a body of the subject under examination.

10. A magnetic resonance imaging apparatus (100) comprising:
the receive coil unit according to any one of claims 1 to 9.
